# EUROPEAN PATENT APPLICATION

(11) **EP 4 775 203 A1**
(43) Date of publication of application: **15.07.2026**
(21) Application number: 24863249.9
(22) Date of filing: 06.09.2024
(51) Int. Cl.: A61K 31/166, A61P 25/28, C07C 233/73

(54) **VANILLIC ACID DERIVATIVE COMPOUND, THERAPEUTIC AGENT COMPOSITION FOR ALZHEIMER'S DISEASE COMPRISING SAME, AND THERAPEUTIC AGENT COMPOSITION FOR NEUROINFLAMMATION**

(30) Priority: 08.09.2023 KR 20230119828; 03.09.2024 KR 20240119204
(71) Applicant: Theranocure Co., Ltd., Daegu 41405 (KR)
(72) Inventor: LEE, Sang Yun, Daegu 42400 (KR)
(74) Representative: Laine IP Oy
(86) International application number: PCT/KR2024/013484
(87) International publication number: WO 2025/053670

(57) **Abstract**

The present invention relates to a vanillic acid derivative compound, which may be formed by a dehydration condensation reaction between vanillic acid and a catecholamine. The vanillic acid derivative compound according to the present invention is characterized in that it can be used as a therapeutic agent for Alzheimer's disease and neuroinflammation.

Also, the present invention relates to a pharmaceutical composition for treating Alzheimer's disease and a pharmaceutical composition for treating neuroinflammation, each comprising the above compound.

## Description

### FIELD

The present invention relates to a compound applicable in the fields of biotechnology and pharmacology, and more particularly to a vanillic acid derivative compound that can be used as a therapeutic agent targeting Alzheimer's disease and neuroinflammation.

### DESCRIPTION OF RELATED ART

Alzheimer's disease is one of the most common neurodegenerative brain disorders worldwide. One of the major pathological features of this disease is the excessive accumulation of amyloid beta protein. Amyloid beta abnormally folds and aggregates, thereby forming plaques between neurons, which impair brain function. In addition, these plaques promote neuroinflammatory responses, thereby further exacerbating neuronal damage.

Conventional therapeutic approaches primarily focus on inhibiting or removing the accumulation of amyloid beta. For example, certain drugs attempt to reduce neuronal damage by decreasing the production of amyloid beta or promoting its clearance from the brain. However, since these approaches target only pathways directly associated with amyloid beta, they have limitations in sufficiently controlling other pathological mechanisms, such as neuroinflammation. Neuroinflammation, which may represent another cause of Alzheimer's disease, is one of the key factors playing an important role in the pathological mechanism of Alzheimer's disease. In particular, various inflammatory factors, including NLRP3, correspond to representative protein complexes that induce neuroinflammation in this process.

Accordingly, there has been increasing recognition that, in order to develop more effective therapeutic agents for Alzheimer's disease, an approach is required that simultaneously inhibits amyloid beta accumulation and targets neuroinflammation. Based on this background, the present invention proposes a novel derivative (Vanillic Acid Derivative, VD) formed by conjugating dopamine to vanillic acid. This VD not only inhibits the aggregation of amyloid beta but also simultaneously suppresses major factors that induce neuroinflammation, thereby demonstrating the potential to delay or prevent the progression of Alzheimer's disease.

Therefore, the present invention seeks to overcome the limitations of existing therapeutic approaches and to present a new paradigm for the treatment of Alzheimer's disease by developing a novel therapeutic agent that simultaneously inhibits neuroinflammation and amyloid beta accumulation. Such a new approach, by targeting multiple pathological pathways including neuroinflammation, may enable a more comprehensive and effective treatment of Alzheimer's disease.

### SUMMARY OF DISCLOSURE

### TECHNICAL PURPOSE

A purpose of the present invention is to provide a vanillic acid derivative compound that is usable as a therapeutic agent for Alzheimer's disease and neuroinflammation.

Another purpose of the present invention is to provide pharmaceutical compositions for treating Alzheimer's disease and neuroinflammation, each comprising the above compound.

### TECHNICAL SOLUTION

In In one aspect, the present invention provides a compound represented by the following Chemical Formula 1:

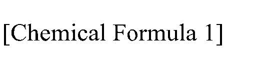

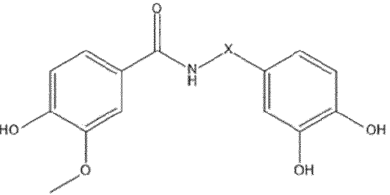

Wherein X is *-(CH₂)ₙ-*, n is an integer independently selected from 0 to 10, and * indicates a bonding site.

Based on the nitrogen (N) position, a structure derived from vanillic acid is located on the left side, and a structure derived from a catecholamine is located on the right side including the nitrogen (N). In the present invention, the term "catecholamine" refers to compounds comprising a catechol structure and an amine group, and the catechol structure means a structure in which two hydroxyl groups are adjacently bonded to a benzene ring. At this time, the benzene ring may further include a chain containing an amine group in addition to the two hydroxyl groups, and the amine group may be coupled to the carboxyl group of vanillic acid through a dehydration condensation reaction to form a structure as represented by Chemical Formula 1. Here, the catecholamine may be dopamine, norepinephrine, epinephrine, or the like.

In the present invention, a linker is present between the amine group of the catechol and the benzene ring, and hereinafter this portion is designated as position X. Here, X may be determined depending on the type of catecholamine-based material that binds to vanillic acid. As long as the compound according to an embodiment of the present invention is formed by the bonding between vanillic acid and a catecholamine as shown in Chemical Formula 1, bonding or removal of bonding acceptable to those skilled in the art is not excluded from the scope of the present invention.

In one embodiment, the compound may be a compound represented by the following Compound 1.

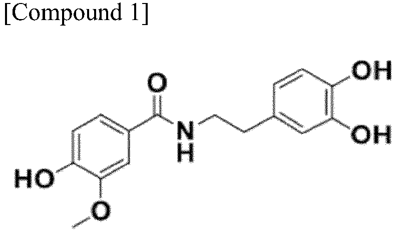

Compound 1 is a compound in which the catecholamine material is selected as dopamine, and an amide bond is formed by a dehydration condensation reaction between dopamine and vanillic acid. Since dopamine has a structure in which two carbon atoms are present in the carbon chain between the nitrogen atom of the amine group and the benzene ring, n is selected as 2, and X becomes *-(CH₂)₂-*.

In one embodiment, the compound may be characterized by targeting a protein complex that induces inflammation. By directly targeting a specific protein complex that causes an inflammatory response, an effect of suppressing or alleviating the inflammatory response can be expected. In particular, in Alzheimer's disease, inflammatory protein complexes are associated with neuronal damage, and targeting such complexes may constitute an important therapeutic strategy capable of inhibiting disease progression.

In one embodiment, the protein complex may comprise amyloid beta. Amyloid beta is one of the major pathological features of Alzheimer's disease, accumulating in the brain to form plaques and interfering with signal transmission between neurons. By targeting a protein complex comprising amyloid beta, the accumulation of amyloid beta and the resulting inflammatory response may be suppressed, which may play an important role in delaying or preventing the progression of Alzheimer's disease.

In one embodiment, the compound may be characterized by targeting a neuroinflammatory factor. Neuroinflammation is one of the important pathological mechanisms of Alzheimer's disease and may induce neuronal damage and cell death. By targeting a factor that induces neuroinflammation, the inflammatory response may be reduced and a neuroprotective effect may be provided, which may serve as an important therapeutic strategy applicable not only to Alzheimer's disease but also to other neurodegenerative diseases.

In one embodiment, the neuroinflammatory factor may comprise NLRP3. NLRP3 is a protein complex that plays an important role in neuroinflammatory responses, and when activated, it may promote the secretion of inflammatory cytokines and cause neuronal damage. By suppressing the inflammatory response through the compound according to the present invention, neuronal damage may be reduced, thereby contributing to delaying the progression of Alzheimer's disease.

In other aspect, the present invention may provide a therapeutic agent for treating Alzheimer's disease comprising the above compound. The novel compound of the present invention may be utilized as a therapeutic agent capable of simultaneously targeting amyloid beta accumulation and neuroinflammation, which are major pathological mechanisms of Alzheimer's disease. It presents the possibility of more effectively inhibiting disease progression than existing therapeutic agents and may provide a new therapeutic option.

In another aspect, the present invention may provide a pharmaceutical composition for treating neuroinflammation comprising the above compound. Neuroinflammation plays an important role in the pathological mechanisms of various neurodegenerative diseases. As a composition capable of effectively suppressing neuroinflammation, it may be applicable not only to Alzheimer's disease but also to other diseases associated with neuroinflammation. This presents a new therapeutic strategy targeting neuroinflammation and may play an important role in the treatment of various neurological disorders.

### TECHNICAL EFFECT

The compound according to the present invention may target both amyloid beta involved in the onset of Alzheimer's disease and neuroinflammatory factors involved in neuroinflammation, thereby exhibiting effects of suppressing both Alzheimer's disease and neuroinflammation.

The therapeutic agent for treating Alzheimer's disease according to the present invention may target and aggregate amyloid beta, thereby contributing to the efficient treatment of Alzheimer's disease.

The pharmaceutical composition for treating neuroinflammation according to the present invention may contribute to exerting a potent anti-inflammatory effect in the treatment of neuroinflammatory diseases.

### BRIEF DESCRIPTION OF DRAWINGS

FIG. 1 shows the chemical structure of the vanillic acid derivative compound according to the present invention.
FIG. 2 shows a chemical reaction mechanism illustrating the method for preparing the vanillic acid derivative compound according to the present invention.
FIG. 3 shows the results of ¹H NMR analysis of the vanillic acid derivative compound according to the present invention.
FIG. 4 shows the results of ¹³C NMR analysis of the vanillic acid derivative compound according to the present invention.
FIG. 5 shows the results of HR-MS analysis of the vanillic acid derivative compound according to the present invention.
FIG. 6 shows the results of purity analysis of the vanillic acid derivative compound according to the present invention.
FIG. 7 shows the results of a cell viability assay of the vanillic acid derivative compound according to the present invention in BV2 cells.
FIG. 8 shows the results of nitric oxide secretion analysis of the vanillic acid derivative compound according to the present invention.
FIG. 9 shows Western blot results for inflammation and inflammation-inducing factors of the vanillic acid derivative compound according to the present invention.
FIG. 10 shows experimental results evaluating protein-ligand interactions (CETSA) of the vanillic acid derivative compound according to the present invention with respect to D1R and D2R.
FIG. 11 shows Western blot results for the CREB factor of the vanillic acid derivative compound according to the present invention.
FIG. 12 shows Western blot results for the NF-κB factor of the vanillic acid derivative compound according to the present invention.
FIG. 13 shows a schematic diagram illustrating the in vivo pathway of the vanillic acid derivative compound according to the present invention.
FIG. 14 shows Western blot bands of the hippocampus of dementia model rats injected with the vanillic acid derivative compound according to the present invention.
FIG. 15 shows Western blot bands of the cerebral cortex of dementia model rats injected with the vanillic acid derivative compound according to the present invention.
FIG. 16 shows a graph illustrating factors affected in the hippocampus and cerebral cortex by the vanillic acid derivative compound according to the present invention.
FIG. 17 shows results of behavioral experiments conducted on dementia model rats to evaluate the effect of the compound according to the present invention on dementia.

### DETAILED DESCRIPTIONS

Hereinafter, embodiments of the present disclosure will be described in detail with reference to the accompanying drawings. The present disclosure may be subjected to various changes and may have various forms. Thus, particular embodiments will be illustrated in the drawings and will be described in detail herein. However, this is not intended to limit the present disclosure to a specific disclosed form. It should be understood that the present disclosure includes all modifications, equivalents, and replacements included in the spirit and technical scope of the present disclosure. While describing the drawings, similar reference numerals are used for similar components. In the accompanying drawings, the dimensions of the structures are shown in an enlarged manner for clarity of the present disclosure.

The terminology used herein is directed to the purpose of describing particular embodiments only and is not intended to be limiting of the present disclosure. As used herein, the singular constitutes "a" and "an" are intended to include the plural constitutes as well, unless the context clearly indicates otherwise. It will be further understood that the terms "comprise", "including", "include", and "including" when used in this specification, specify the presence of the stated features, integers, operations, elements, and/or components, but do not preclude the presence or addition of one or more other features, integers, operations, elements, components, and/or portions thereof.

Unless otherwise defined, all terms including technical and scientific terms used herein have the same meaning as commonly understood by one of ordinary skill in the art to which this inventive concept belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

Alzheimer's disease is one of the most common neurodegenerative disorders worldwide, primarily occurring in the elderly and known as a major cause of dementia. One of the principal pathological features of this disease is the excessive expression and aggregation of amyloid beta protein. Such excessive expression and aggregation of amyloid beta protein result in neuronal damage and cell death, ultimately causing major symptoms of Alzheimer's disease, such as memory decline and cognitive impairment. In addition, Alzheimer's disease does not occur solely due to the accumulation of amyloid beta, but neuroinflammation also plays an important role in disease progression. In particular, inflammatory factors such as the NLRP3 inflammasome may induce neuroinflammation and further accelerate neuronal damage. Such neuroinflammation plays a central role in the pathological mechanism of Alzheimer's disease, and a therapeutic strategy targeting these factors simultaneously is required.

In order to address these issues, the present invention has developed a vanillic acid derivative compound according to the present invention. The vanillic acid derivative compound according to the present invention is a novel substance that can be used as a therapeutic agent for both Alzheimer's disease and neuroinflammation. As one embodiment, a novel compound was developed by conjugating dopamine to vanillic acid, which is hereinafter abbreviated as VD (Vanillic-Dopamine). VD is intended to simultaneously target amyloid beta and neuroinflammatory factors, thereby inhibiting and treating the progression of Alzheimer's disease. The purpose of the present invention is to overcome the limitations of existing therapeutic agents and to provide a more effective treatment by simultaneously targeting various pathological factors associated with Alzheimer's disease and neuroinflammation through VD.

Such an approach presents a new paradigm for the treatment of Alzheimer's disease and suggests the possibility of delaying or preventing disease progression. The development of VD is expected to open a new avenue for Alzheimer's disease treatment by targeting not only the suppression of amyloid beta accumulation but also the fundamental causes of neuroinflammation.

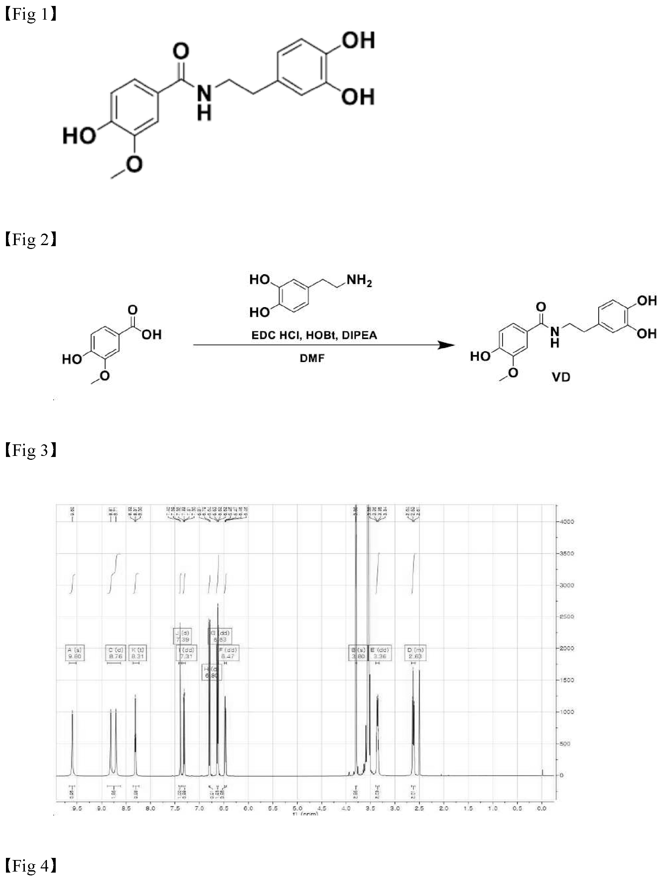

FIG. 1 shows the chemical structure of the vanillic acid derivative compound according to one embodiment of the present invention. The present invention is achieved through a dehydration condensation reaction between vanillic acid and a catecholamine, and the compound shown in FIG. 1 uses dopamine as the catecholamine material. The chemical structures of vanillic acid and dopamine are illustrated below.

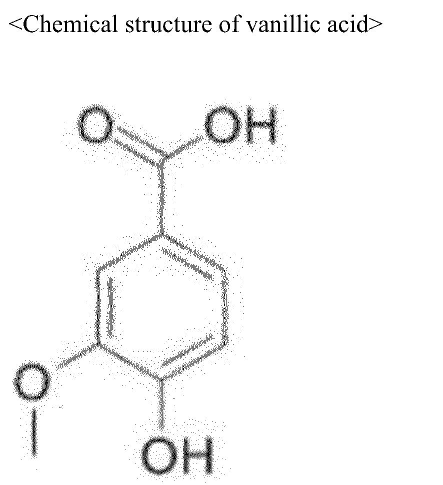

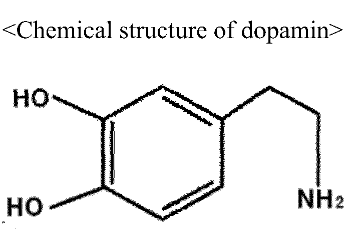

In the compound shown in FIG. 1, a nitrogen atom (N) is present at the center, which is derived from the amine group of dopamine. Based on this nitrogen atom, a derivative structure is formed on the left side in which the -OH group of the carboxyl group of vanillic acid is removed and the nitrogen atom of dopamine is bonded thereto. Including this nitrogen atom, on the right side, one hydrogen atom of the amine group of dopamine is removed and bonded to vanillic acid. This results from a dehydration condensation reaction between the - OH group of the carboxyl group of vanillic acid and the -H group of the amine group of dopamine, thereby forming an intermolecular amide bond (-CONH-) between the two molecules. Through this reaction, a new chemical structure may be formed.

In FIG. 1, the nitrogen atom and the benzene ring are connected by two carbon atoms, which results from conjugating dopamine to vanillic acid. If a catecholamine-based material other than dopamine is used, the structure between the nitrogen atom and the benzene ring may vary, and this portion is hereinafter designated as position X. As long as the compound according to an embodiment of the present invention is formed by the bonding between vanillic acid and a catecholamine as represented by Chemical Formula 1, bonding or removal of bonding acceptable to those skilled in the art is not excluded from the scope of the present invention.

FIG. 2 illustrates a chemical reaction mechanism showing the method for preparing the vanillic acid derivative compound according to the present invention, which will be described in detail in the following Examples.

### (Preparation Method)

A method for preparing the compound according to the present invention includes obtaining a vanillic acid derivative compound by dissolving vanillic acid, a catecholamine-based material, a coupling reagent, an acid catalyst, a base catalyst, and an amide bond formation promoter in an organic solvent and allowing them to react. In one embodiment, the phenylalkylamine-based material may include dopamine. In one embodiment, the organic solvent may include dimethylformamide (DMF). In the present invention, the coupling reagent is a substance used to bind two molecules; the acid catalyst is a substance that promotes the reaction by interacting with the coupling reagent to provide protons; the base catalyst is a substance that promotes the reaction by removing protons during the reaction; and the amide bond formation promoter is a substance that increases efficiency in forming an amide bond and reduces side reactions. In one embodiment, the coupling reagent may include 1-ethyl-3-(3-dimethylaminopropyl)carbodiimide (EDC), the acid catalyst may include hydrochloric acid (HCl), the base catalyst may include N,N-diisopropylethylamine, and the amide bond formation promoter may include 1-hydroxybenzotriazole (HOBt).

A specific preparation procedure is as follows. First, vanillic acid (18.97 mmol) was dissolved in 50 mL of dimethylformamide, and hydroxybenzotriazole (20.87 mmol) and 1-ethyl-3-(3-dimethylaminopropyl)carbodiimide (20.87 mmol) were added and stirred at 0°C for 30 minutes. Thereafter, dopamine (18.97 mmol) and N,N-diisopropylethylamine (37.94 mmol) were added and stirred at room temperature for 24 hours, and the product was extracted using dichloromethane, water, and brine. After evaporating the extracted solvent to dryness, the residue was dissolved in methanol and separated by flash chromatography using water/acetonitrile. After removing the solvent from the collected product and vacuum drying, the vanillic acid derivative compound VD according to the present invention was obtained. The yield was approximately 29%.

Hereinafter, an experimental example for confirming the finally synthesized compound, Gd-DO3A-SPA, will be described.

### (Experimental Example) Structural Analysis and Purity Analysis of Final Compound VD

In order to confirm whether the vanillic acid derivative compound VD according to the present invention corresponds to the expected structure and to verify that the synthesis was successfully completed, structural analysis of VD was performed. For structural analysis of VD, the structural characteristics of the compound were thoroughly examined by ¹H NMR, ¹³C NMR, and HR-MS analyses.

FIG. 3 shows the results of ¹H NMR analysis of the vanillic acid derivative compound according to the present invention. In the compound, hydrogen atoms at specific positions exhibit different chemical shifts (δ) depending on their chemical environments. The peak at δ = 9.60 (s, 1H) appears to correspond to the -NH- hydrogen atom of the amide bond. It appears as a singlet, indicating that there are no adjacent hydrogen atoms around this hydrogen. The peaks at δ = 8.76 (d, J = 52.1 Hz, 2H), δ = 8.31 (t, J = 5.6 Hz, 1H), δ = 7.39 (d, J = 2.0 Hz, 1H), δ = 7.31 (dd, J = 8.2, 2.0 Hz, 1H), δ = 6.80 (d, J = 8.2 Hz, 1H), δ = 6.63 (dd, J = 9.5, 5.0 Hz, 2H), and δ = 6.47 (dd, J = 8.0, 2.0 Hz, 1H) indicate that the hydrogen atoms on the aromatic rings are undergoing spin-spin coupling with adjacent hydrogen atoms. It is presumed that these correspond to the aromatic ring hydrogens present in vanillic acid and dopamine. The peak at δ = 3.80 (s, 3H) corresponds to the hydrogen atoms of the methoxy group (-OCH₃), appearing as a singlet. The peak at δ = 3.36 (dd, J = 14.8, 6.0 Hz, 2H) appears as a doublet of doublets (dd) and likely corresponds to a methylene group (-CH₂-) located near the amide bond.

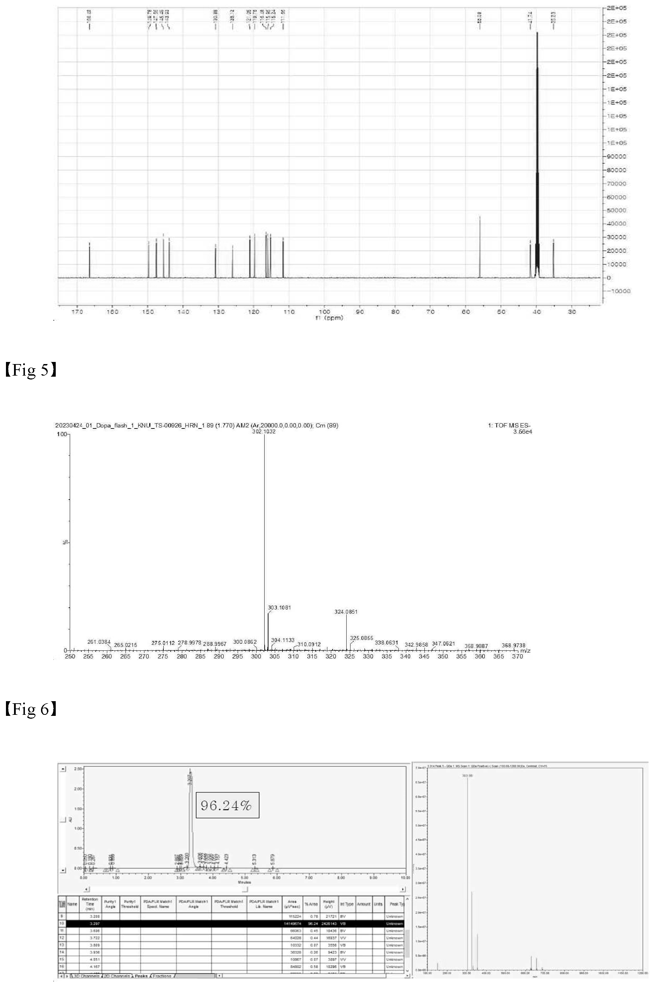

FIG. 4 shows the results of ¹³C NMR analysis of the vanillic acid derivative compound according to the present invention. The peak at δ = 166.42 ppm corresponds to the carbonyl carbon of the amide bond and exhibits a high chemical shift value. The peaks at δ = 149.78 ppm, 147.56 ppm, and 145.49 ppm are highly likely to correspond to carbon atoms of the catechol structure within the aromatic ring. The peak at δ = 143.93 ppm appears to correspond to a carbon atom in the aromatic ring of the vanillic acid structure. The peaks at δ = 130.88 ppm and 126.12 ppm correspond to other carbon atoms in the aromatic ring, which may be carbons bonded to different substituents. The peaks at δ = 121.05 ppm, 119.76 ppm, 116.48 ppm, 115.96 ppm, 115.24 ppm, and 111.66 ppm also correspond to aromatic carbon atoms, each potentially bonded to substituents at different positions. The peak at δ = 56.08 ppm appears to correspond to the carbon atom of the methoxy group (-OCH₃). The peaks at δ = 41.74 ppm and 35.23 ppm correspond to carbon atoms of methylene groups (-CH₂-), each connected to the amide bond at their respective positions.

FIG. 5 shows the results of HR-MS analysis of the vanillic acid derivative compound according to the present invention. Through the HR-MS analysis, the molecular weight of the prepared compound can be determined. The measured molecular weight [M+H]⁺ was 302.1028, which exactly matched the theoretically calculated molecular weight of 302.1028. This indicates that the synthesized compound accurately reflects the intended molecular structure of VD, namely C₁₆H₁₇N₁O₅.

The results of the ¹H NMR, ¹³C NMR, and HR-MS analyses of the fourth powder shown in FIGS. 2-5 confirmed that the synthesized compound was consistent with the expected chemical structure. The ^1H NMR and ^13C NMR spectra showed signals expected from a sinapic acid derivative and a DO3A backbone, and the HR-MS analysis demonstrated that the molecular weight closely matched the expected structure. Through these analyses, it was confirmed that the fourth powder was successfully synthesized.

FIG. 6 shows the results of purity analysis of the vanillic acid derivative compound according to the present invention. A major peak was observed at approximately 3.297 minutes, indicating that the compound was detected as the principal component when passing through the HPLC column. Other peaks may correspond to impurities or reaction by-products, and the purity and degree of purification of the compound may be evaluated in consideration of these peaks. The table records the retention time (RT), area, and height of each peak, thereby providing information regarding signal intensity and purity assessment. In this analysis, the purity was determined based on the proportion of the major peak relative to the total peaks, and as a result, the purity was confirmed to be 96.24%.

**[Table 1]**

| | the theoretically calculated % | | | | the experimentally measured % | | | |
|---|---|---|---|---|---|---|---|---|
| | C | H | N | O | C | H | N | O |
| VD+H₂O | 59.81 | 5.96 | 4.36 | 29.87 | 59.60 | 5.93 | 4.48 | 29.61 |

Table 1 shows the results of elemental analysis (EA) performed to determine the content of the major elements in VD. If the deviation between the theoretical values and the experimental values is within ±0.4%, the analysis may be considered accurate. The deviations observed for each element were 0.21% for carbon, 0.03% for hydrogen, 0.12% for nitrogen, and 0.26% for oxygen, all of which fall within the acceptable error range. Based on the above analyses, it was confirmed that the VD compound was synthesized with the expected structure and possesses high purity and an accurate molecular weight. This may be regarded as verification of the successful synthesis and characterization of the VD compound.

Hereinafter, evaluation examples describing methods for assessing the performance of the synthesized material and the results thereof will be explained.

### (Evaluation Example 1) Cell Viability Assay Results

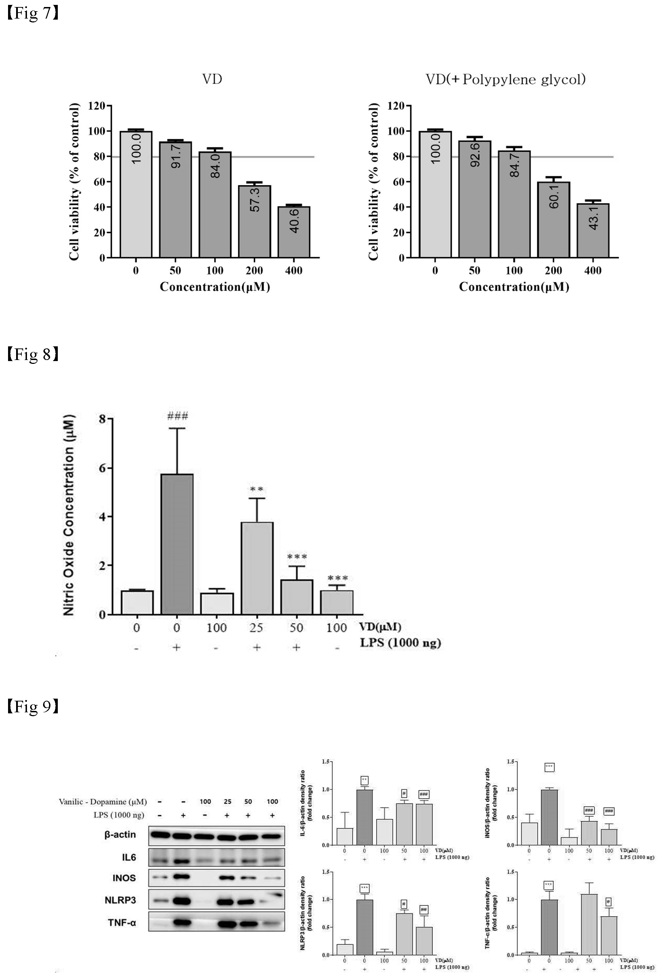

FIG. 7 shows the results of a cell viability assay of the vanillic acid derivative compound according to the present invention in BV2 cells. The cytotoxicity of the synthesized VD was evaluated through a cell viability assay. BV2 cells (normal mouse brain-derived cells) were used. The cells were cultured in a growth medium containing DMEM supplemented with 10% FBS (fetal bovine serum). The medium further contained 1% antibiotic-antimycotic solution, 1% insulin-transferrin-selenium, and 40 ng/mL dexamethasone, and the cells were incubated at 37°C in a humidified atmosphere containing 5% CO₂. VD dissolved in saline was treated to the cells at various concentrations (0.5-400 mM). After maintaining the cells under serum-free conditions for 24 hours, subsequent incubation for 48 hours was performed using RPMI 1640 medium containing 10% FBS and drugs (cisplatin and WP1130). Thereafter, fresh complete medium containing Cell Counting Kit-8 (CCK-8) solution was added to the plate and incubated for 3 hours. Cell viability was measured by absorbance at 450 nm using a SpectraMax^{®} i3 (Molecular Devices, CA, U.S.).

The left graph (VD) shows how cell viability changes according to the concentration of VD. At 0 µM (i.e., untreated control), cell viability was set at 100%, indicating normal survival of all cells. At higher concentrations (200 µM or more), slight cytotoxicity was observed, resulting in decreased cell viability. However, at lower concentrations (0-100 µM), VD exhibited little to no cytotoxicity, and cell viability remained high. The right graph (VD + additive) shows cell viability when propylene glycol (PG), an additive used to improve the solubility of VD, was added. Cell viability was 92.6% at 0 µM, 84.7% at 100 µM, 60.1% at 200 µM, and 43.1% at 400 µM, showing a pattern similar to VD alone. This indicates that the combined use of VD and the additive (PG) does not significantly differ from VD treatment alone in terms of cell viability. These results provide important information for evaluating the effect of VD on cell survival and indicate that VD exhibits no or minimal cytotoxicity at appropriate concentrations. Such data may be valuable in the potential development of VD as a therapeutic agent.

### (Evaluation Example 2) Nitric Oxide (NO) Secretion Analysis Results

FIG. 8 shows the results of nitric oxide (NO) secretion analysis of the vanillic acid derivative compound according to the present invention. This evaluation example was conducted to assess the effect of VD on nitric oxide (NO) secretion. Nitric oxides such as NO are inflammatory mediators that increase during inflammatory responses or infection and serve as important indicators of inflammation severity. By determining whether VD can inhibit NO secretion, the anti-inflammatory effect of VD was evaluated. After stabilizing BV2 cells, the cells were treated with LPS for 2 hours, followed by the addition of VD and incubation for 12 hours. The supernatant was collected from the cultured cells and centrifuged at 5,000 rpm for 2 minutes at 4°C. The analysis was performed using a Griess reagent kit, and the concentration of nitric oxide was calculated by measuring absorbance at 540 nm.

Since the cell viability experiment shown in FIG. 7 confirmed that VD exhibited no cytotoxicity up to 100 µM, the subsequent experiments were conducted using concentrations up to 100 µM. A high level of NO secretion was observed in the LPS-treated group; however, in the groups treated with both LPS and VD, lower NO secretion was observed at all tested concentrations compared to the LPS-only group. These results demonstrate that VD effectively inhibits NO secretion in LPS-induced inflammatory responses. This suggests that VD possesses potential as an anti-inflammatory agent and may regulate inflammatory responses by reducing the production of the inflammatory mediator NO. Such findings provide important evidence for evaluating the potential use of VD as a therapeutic agent for inflammatory diseases.

### (Evaluation Example 3) In Vitro Results on the Therapeutic Effect of VD

This evaluation example was conducted to investigate the fundamental effects of the drug at the cellular level. The purpose was to preliminarily verify the potential therapeutic effect of VD and to understand its safety, efficacy, and mechanism of action before proceeding to more complex in vivo experiments or clinical studies.

FIG. 9 shows the Western blot results for inflammatory and pro-inflammatory factors of the vanillic acid derivative compound according to the present invention. This in vitro experiment was performed to evaluate the ability of VD to suppress inflammatory responses. After extracting proteins from treated cells, the expression levels of inflammatory factors such as IL-6, iNOS, NLRP3, and TNF-α were analyzed by Western blotting. β-actin was used as an internal control for protein quantification. As a result, the intensity of NLRP3, an inflammatory factor, was increased in the LPS-only treated group, whereas the intensity was effectively reduced in the VD-treated group. Pro-inflammatory factors such as IL-6, iNOS, and TNF-α showed the same pattern as NLRP3. Accordingly, it may be concluded that VD affects both inflammatory and pro-inflammatory factors. These results suggest that VD may regulate inflammatory responses and be useful in the treatment of inflammatory diseases.

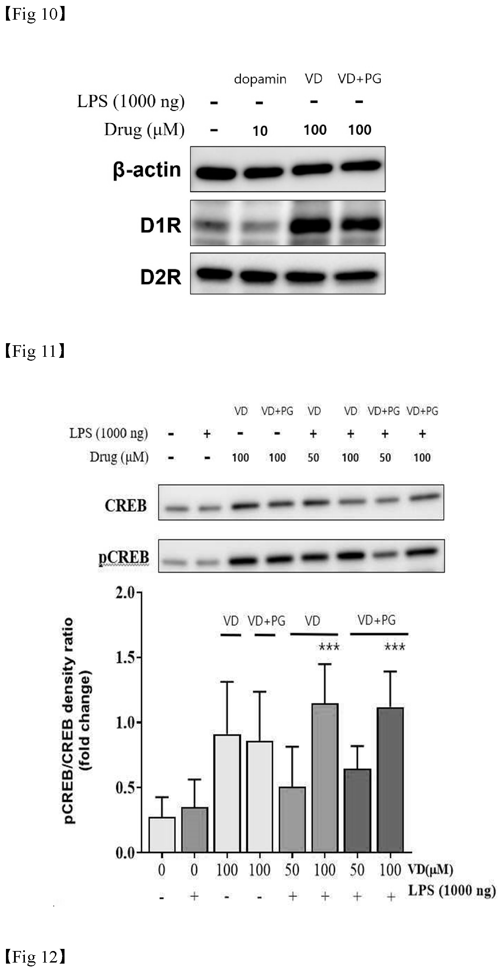

FIG. 10 shows the experimental results evaluating protein-ligand interactions (CETSA) of the vanillic acid derivative compound with respect to D1R and D2R. This experiment was conducted to identify the receptor targeted by VD. Dopamine receptors (D1-D5) are present on the membranes of neurons, microglia, and astrocytes. The study aimed to determine through which pathway VD exerts its anti-inflammatory effects. Since dopamine receptors are broadly classified into the D1-like and D2-like families, representative dopamine receptor 1 (D1R) and dopamine receptor 2 (D2R) were evaluated using CETSA. The results showed no significant change in D2R, whereas a change was observed in D1R. Although it is unclear whether VD acts as an agonist or antagonist, the results indicate that VD at least affects dopamine receptor 1.

These findings suggest that VD may exert anti-inflammatory effects by acting on the D1R dopamine receptor. Since no effect was observed on D2R, VD is likely to selectively target D1R. Accordingly, VD may possess a mechanism for regulating or suppressing inflammatory responses through D1R. This experiment provides important insight into the mechanism of action of VD.

FIG. 11 shows the Western blot results for the CREB factor of the vanillic acid derivative compound according to the present invention. This experiment was performed to evaluate how VD regulates the CREB factor, which plays an important role in inflammatory signaling pathways. CREB (cAMP response element-binding protein) is a transcription factor that regulates various intracellular signaling pathways. When activated, CREB plays an important role in suppressing the NF-κB pathway. Since NF-κB is one of the major mediators of inflammatory responses, activation of CREB is closely associated with inflammation suppression. After inducing an inflammatory response in cells by treating them with LPS (1000 ng/mL), VD was subsequently administered at concentrations of 10 µM, 50 µM, and 100 µM. A group treated with both VD and the additive (propylene glycol, PG) was also included. Western blot analysis was then performed to examine the expression levels of CREB and its activated form, pCREB. β-actin was used as an internal control for protein quantification.

Based on the Western blot results and corresponding graphs, a significant increase in the pCREB/CREB ratio was observed at 100 µM VD regardless of the presence of the additive. This indicates that VD activates CREB. These results suggest that VD may suppress the NF-κB pathway and regulate inflammatory responses by activating the CREB factor. Activation of CREB plays a crucial role in inflammation suppression, and VD may exert its effects through the CREB pathway. Accordingly, VD possesses potential therapeutic efficacy in inflammatory diseases and may exhibit anti-inflammatory effects through modulation of the CREB signaling pathway.

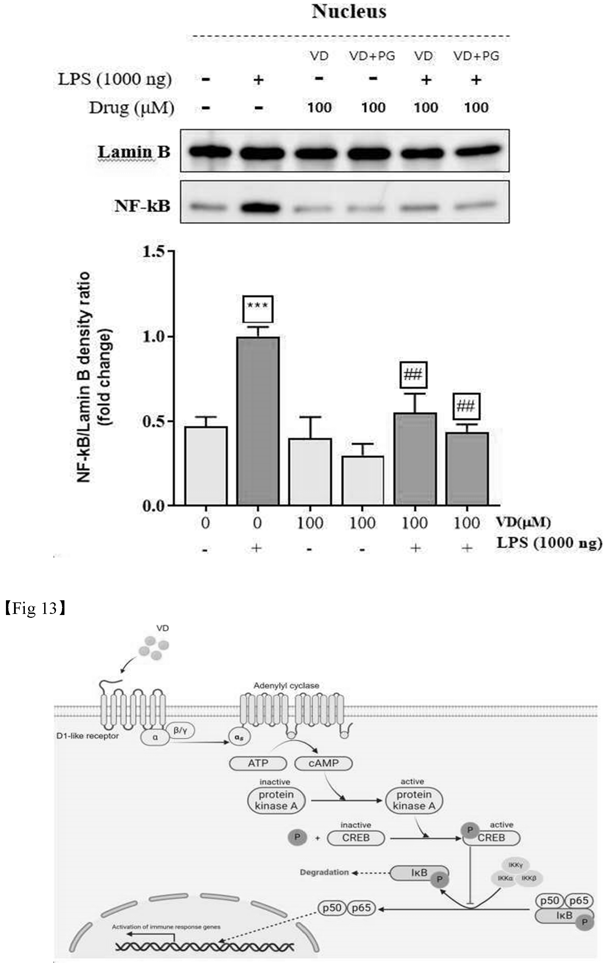

FIG. 12 shows the Western blot results for the NF-κB factor of the vanillic acid derivative compound according to the present invention. This experiment was conducted to evaluate how VD regulates the NF-κB pathway, which plays a critical role in inflammatory responses. NF-κB is a key transcription factor that regulates inflammatory responses, and when activated within cells, it promotes the expression of inflammatory cytokines. Therefore, inhibition of NF-κB activation may play an important role in suppressing inflammation. CREB acts upstream of the NF-κB pathway, and activation of CREB is associated with inhibition of the NF-κB pathway. Cells were treated with LPS (1000 ng/mL) to activate the NF-κB pathway and induce an inflammatory response, followed by treatment with VD at a concentration of 100 µM. A group treated with both VD and the additive (propylene glycol, PG) was also included for comparison. Western blot analysis was performed to examine the expression of pNF-κB, the activated form of NF-κB, and Lamin B was used as a control for nuclear protein quantification. In the group treated with 100 µM VD, activation of NF-κB was significantly reduced. These results suggest that VD suppresses the NF-κB pathway and thereby inhibits inflammatory responses.

The above experimental results demonstrate that VD can regulate inflammatory responses by inhibiting the NF-κB pathway. Since NF-κB is a major transcription factor that promotes the expression of inflammatory cytokines, inhibition of the NF-κB pathway by VD may represent an important therapeutic strategy in inflammatory diseases. Accordingly, VD exhibits potential as a therapeutic agent for inflammation suppression.

FIG. 13 schematically illustrates the mechanism of action of VD based on the foregoing experimental results. This diagram explains how VD regulates inflammatory responses within cells.

The specific mechanism is as follows. VD binds to the D1R dopamine receptor and transmits intracellular signals. The D1R receptor is a G protein-coupled receptor that activates adenylyl cyclase, thereby promoting the production of cAMP. Increased cAMP activates protein kinase A (PKA), and activated PKA phosphorylates and activates CREB (cAMP response element-binding protein). Activated CREB suppresses the NF-κB pathway. Since NF-κB is a key transcription factor that induces the expression of inflammatory cytokines, activation of CREB by VD leads to inhibition of NF-κB and a consequent reduction in inflammatory responses. Thus, VD may reduce the expression of inflammatory factors by suppressing NF-κB activation through the CREB pathway. This suggests that VD may be useful in the treatment of inflammatory diseases by regulating cytokine-related factors through this signaling pathway.

### (Evaluation Example 4) In Vivo Results on the Therapeutic Effect of VD

This evaluation example was conducted to assess the effect of VD (vanillic acid-dopamine derivative) on the expression of specific proteins when administered to a 12-month-old Alzheimer's disease mouse model (5xFAD).

A total of 13 twelve-month-old 5xFAD mice were used in this experiment. The experiment was divided into three groups: (1) a normal wild-type group, (2) a dementia-induced group administered saline (5xFAD + saline), and (3) a dementia-induced group administered VD (5xFAD + VD). Each group received intravenous (IV) injections of the respective substance six times over a total period of 20 days. After completion of the treatment, the brains were harvested from the mice, and hippocampal tissue was isolated for further analysis. Protein expression of specific factors was evaluated using the hippocampal tissue. To extract proteins, the tissue was homogenized in RIPA buffer and centrifuged at 13,000 rpm for 15 minutes at 4°C. The supernatant was collected, and protein concentration was measured using a BCA protein assay kit. Equal amounts of protein were loaded onto 8.5% SDS-PAGE gels and transferred to an Immobilon-P membrane. The membranes were blocked with 3% bovine serum albumin (BSA) in Tris-buffered saline containing 0.05% Tween-20 (TBS-T) for 3 hours at room temperature. Thereafter, diluted primary antibodies were incubated overnight at 4°C in 3% BSA. The membranes were washed three times and then incubated with secondary antibodies for 1 hour. Western blot bands were detected using PicoEPD Western Reagent and SuperSignal^{™} West Femto Maximum Sensitivity Substrate, and images were captured using a chemiluminescent western imaging system. The obtained images were analyzed using ImageJ software.

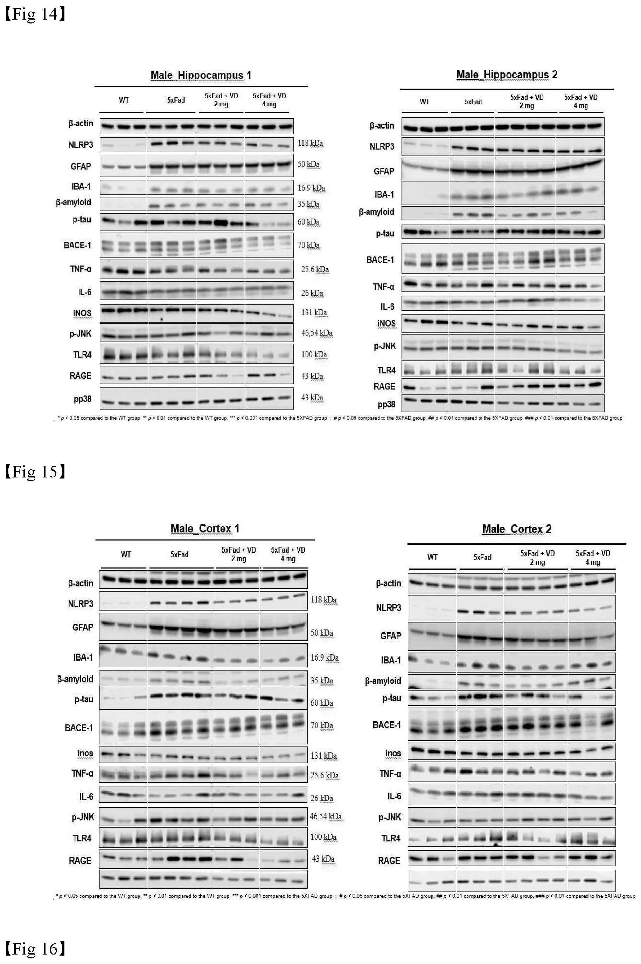

FIGS. 14 and 15 show Western blot results analyzing changes in protein expression in the hippocampus and cerebral cortex, respectively, after treatment with VD (vanillic acid-dopamine derivative). These experiments were conducted using Alzheimer's disease model mice (5xFAD), and the expression levels of various inflammatory and neurodegeneration-related proteins were compared to evaluate the effect of VD. Both FIGS. 14 and 15 were conducted in two independent sets, with the left panels showing the results of the first set and the right panels showing the results of the second set.

The results of FIGS. 14 and 15 demonstrate that VD can suppress inflammatory and neurodegenerative changes in the hippocampus and cerebral cortex of Alzheimer's model mice. Specifically, VD reduced the expression of inflammatory cytokines and neurotoxic proteins (such as β-amyloid and phosphorylated tau), suggesting that VD possesses the potential to inhibit the pathological progression of Alzheimer's disease.

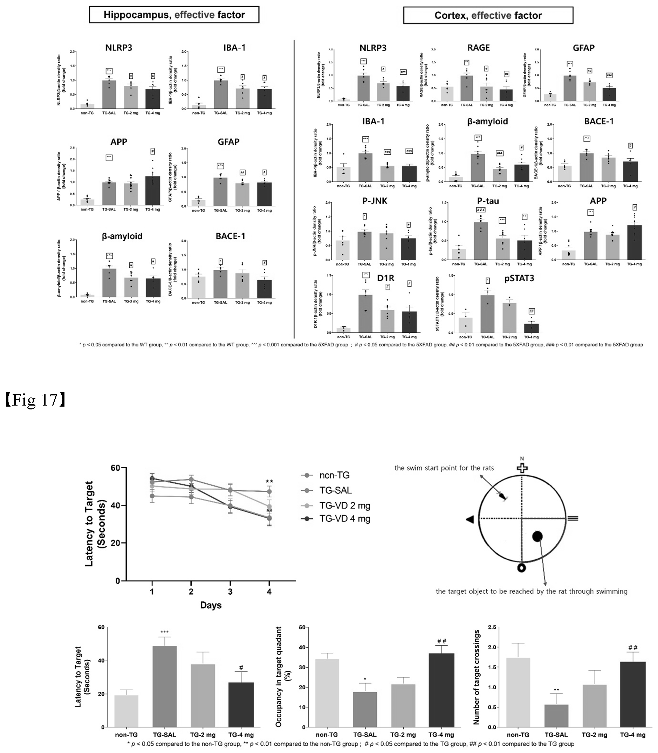

FIG. 16 shows Western blot results illustrating changes in the expression of various inflammation- and neurodegeneration-related factors in the hippocampus and cerebral cortex following treatment with VD (vanillic acid-dopamine derivative). This figure quantitatively evaluates the anti-inflammatory and neuroprotective effects of VD in Alzheimer's disease model mice (5xFAD). For this purpose, VD was administered to dementia model mice at two doses, 2 mg/kg and 4 mg/kg. The inflammatory and neurodegeneration-related factors analyzed in this experiment were as follows. NLRP3 is a protein complex acting as an inflammatory factor that plays an important role in regulating immune responses and inducing inflammation. BACE-1 (Beta-site Amyloid Precursor Protein Cleaving Enzyme 1) is an enzyme that cleaves amyloid precursor protein (APP) to generate β-amyloid. β-amyloid is one of the primary causative substances of Alzheimer's disease, and its accumulation is considered a key pathological feature of the disease. IBA-1 is a marker of microglial activation, indicating activation of microglia, which play a crucial role in immune and inflammatory responses in the brain. GFAP is a marker of astrocyte activation, and increased GFAP expression indicates activation of astrocytes. When astrocytes and microglia are activated, they secrete pro-inflammatory cytokines, thereby promoting inflammatory responses.

The experimental results showed that VD suppressed the expression of inflammation- and neuroinflammation-related factors such as NLRP3, BACE-1, IBA-1, and GFAP in both the hippocampus and cerebral cortex. Considering that Alzheimer's-related factors such as β-amyloid and phosphorylated tau were reduced, along with decreased levels of inflammatory markers, it can be inferred that VD exerts beneficial effects on Alzheimer's disease pathology.

FIG. 17 shows the results of a water maze experiment evaluating the effect of VD (vanillic acid-dopamine derivative) treatment on cognitive function in a dementia mouse model (5xFAD). Latency to Target represents the time required to reach the target platform. As dementia progresses, cognitive function declines, leading to increased latency. In VD-treated dementia mice, the time required to reach the target was shorter compared to untreated dementia mice, with the most significant improvement observed in the 4 mg/kg VD-treated group.

Occupancy in Target Quadrant refers to the extent to which the mice reached or remained in the quadrant containing the target. Compared to untreated dementia mice, the VD-treated groups, particularly the 4 mg/kg group, showed a higher occupancy rate. This suggests that VD improved spatial memory in the dementia mice. Number of Target Crossings represents the number of times the mice crossed the location of the target platform. The VD-treated group crossed the target area more frequently, with notably higher values in the 4 mg/kg group compared to untreated dementia mice. This indicates that VD improved learning and memory ability in the dementia model.

In conclusion, these experimental results demonstrate that VD has the potential to improve cognitive function in dementia model mice. VD-treated mice exhibited better performance in cognitive tests, with the most pronounced effects observed at a dose of 4 mg/kg.

While the present invention has been described with reference to preferred embodiments thereof, those skilled in the art will understand that various modifications and changes may be made without departing from the spirit and scope of the present invention as set forth in the appended claims.

## Claims

1. A compound represented by the following Chemical Formula 1: 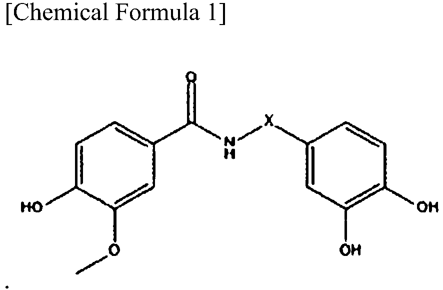 wherein, X is *-(CH₂)ₙ-*, n is an integer independently selected from 0 to 10, and * indicates a bonding site.

2. The compound of claim 1, wherein the compound is represented by the following Compound 1:

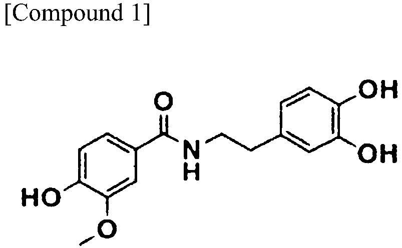

3. The compound of claim 1, wherein the compound is **characterized by** targeting a protein complex that induces inflammation.

4. The compound of claim 3, wherein the protein complex comprises amyloid beta.

5. The compound of claim 1, wherein the compound is **characterized by** targeting a neuroinflammatory factor.

6. The compound of claim 5, wherein the neuroinflammatory factor comprises NLRP3.

7. A pharmaceutical composition for treating Alzheimer's disease, comprising the compound according to any one of claims 1 to 6.

8. A pharmaceutical composition for treating neuroinflammation, comprising the compound according to any one of claims 1 to 6.
